Europäisches Patentamt

European Patent Office

Office européen des brevets

(11) Veröffentlichungsnummer: **0 324 350 B1**

(12) **EUROPÄISCHE PATENTSCHRIFT**

(45) Veröffentlichungstag der Patentschrift: **10.11.93**

(21) Anmeldenummer: **89100038.2**

(22) Anmeldetag: **03.01.89**

(51) Int. Cl.⁵: **C12N 15/39**, C12N 7/00, A61K 39/285

(54) **Rekombinantes Vaccinia-Virus MVA.**

(30) Priorität: **12.01.88 CH 85/88**

(43) Veröffentlichungstag der Anmeldung:
**19.07.89 Patentblatt 89/29**

(45) Bekanntmachung des Hinweises auf die
Patenterteilung:
**10.11.93 Patentblatt 93/45**

(84) Benannte Vertragsstaaten:
**AT BE CH DE ES FR GB IT LI LU NL SE**

(56) Entgegenhaltungen:
**EP-A- 0 083 286**
**EP-A- 0 110 385**
**EP-A- 0 198 328**
**EP-A- 0 261 940**
**CH-A- 568 392**

**Biotechnology & Genetic Engineering Reviews, Band 2, Oktober 1984 (Newcastle upon Tyne, GB), G.L. Smith et al "Recombinant Vaccinia viruses as New Live vaccines", Seiten 383-407**

(73) Patentinhaber: **F. HOFFMANN-LA ROCHE AG**
**Postfach 3255**
**CH-4002 Basel(CH)**

(72) Erfinder: **Altenburger, Werner, Dr.**
**Esterliweg 135**
**CH-4125 Riehen(CH)**

(74) Vertreter: **Mezger, Wolfgang, Dr. et al**
**Grenzacherstrasse 124**
**Postfach 3255**
**CH-4002 Basel (CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Vaccinia-Viren gehören zur Familie der Pockenviren. Gewisse Vaccinia-Virusstämme wurden während vieler Jahre als Lebendimpfstoff zur Pockenschutzimpfung eingesetzt, so zum Beispiel der Stamm Elstree des Lister-Institutes in England. Wegen der Komplikationen, die sich aus der Impfung ergeben können (vergleiche Schär. Zeitschr. für Präventivmedizin 18, 41-44 [1973]), werden heute, nachdem die WHO im Jahre 1980 die Ausrottung der Pocken deklariert hatte, nur noch stark gefährdete Personen gegen Pocken geimpft.

Vaccinia-Viren sind in neuerer Zeit auch als Vektoren für fremde Antigene verwendet worden (Smith et al., Biotechnology and Genetic Engineering Reviews 2, 383-407 [1984]). Dabei werden mit Hilfe der DNA-Rekombinationstechnik DNA-Sequenzen (Gene), die für fremde Antigene codieren, in das Genom der Vaccinia-Viren eingeführt. Erfolgt die Integration des Gens an einem Ort der viralen DNA, der für den Lebenszyklus des Virus nicht essentiell ist, so ist es möglich, dass das neu entstandene rekombinante Vaccinia-Virus infektiös ist, d.h. fähig ist, fremde Zellen zu infizieren und dabei die integrierte DNA-Sequenz zu exprimieren (vergleiche Europäische Patentanmeldungen mit den Publikations-Nummern 83 286 [publiziert am 6.7.1983] und 110 385 [publiziert am 13.6.1984]). Die so hergestellten rekombinanten Vaccinia Viren können einerseits als Lebendimpfstoffe zur Prophylaxe von Infektionen, die durch diese Antigene verursacht werden, oder andererseits zur Herstellung von heterologen Proteinen in eukaryotischen Zellen, verwendet werden. Die in der Literatur beschriebenen rekombinanten Vaccinia-Viren basieren meist auf dem Stamm WR. Es ist andererseits bekannt, dass dieser Stamm eine hohe Neurovirulenz aufweist und daher für den Einsatz beim Menschen und bei Tieren wenig geeignet ist (Morita et al., Vaccine 5, 65-70 [1987]).

Schon seit längerer Zeit sind aber Virusstämme bekannt, die speziell gezüchtet wurden, um unerwünschte Nebenwirkungen zu vermeiden. So gelang es, durch serielle Passagen des Vaccinia-Virusstammes Ankara (CVA) auf Hühnerfibroblasten, ein modifiziertes Vaccinia Virus Ankara (MVA) zu züchten (Schweiz. Patentschrift Nr. 568 392). Dieses modifizierte Vaccinia Virus Ankara weist nur eine geringe Virulenz auf, d.h. es hat, wenn es bei Impfungen verwendet wird, keine Nebenwirkungen zur Folge. Es eignet sich daher besonders zur Erstimpfung von immunabwehrgeschwächten Impflingen. Die ausgezeichneten Eigenschaften des Stammes MVA sind durch eine grosse Zahl klinischer Versuche belegt (Mayr et al., Zbl. Bakt. Hyg. I, Abt. Org. B 167, 375-390 [1978], Stickl et al., Dtsch. med. Wschr. 99, 2386-2392 [1974]).

Es wurde daher nach einer Möglichkeit gesucht, fremde Gene durch DNA-Rekombination in den Vaccinia-Virusstamm MVA einzuführen. Da das Genom des MVA, ausser an der Rekombinationsstelle, nicht verändert werden sollte, musste ein Verfahren verwendet werden, das diesem Erfordernis genügte. Da das Thymidinkinasegen (TK-Gen) des MVA für den Lebenszyklus des Virus nicht von grundlegender Bedeutung ist, wurde die fremde DNA-Sequenz in die DNA des Vaccinia-TK-Gens rekombiniert.

Die vorliegende Erfindung betrifft deshalb rekombinante Vaccinia-Viren MVA, die ein Gen enthalten, das für ein fremdes Antigen, vorzugsweise eines pathogenen Agens codiert, sowie Vakzine, die ein solches Virus in einer physiologisch akzeptablen Form enthalten. Die Erfindung betrifft ferner Verfahren zur Herstellung solcher rekombinanten Vaccinia Viren MVA oder Vakzine, sowie die Verwendung dieser Vakzine zur Prophylaxe von Infektionen, die durch solche Antigene bzw. pathogenen Agentien hervorgerufen werden.

Die rekombinanten Vaccinia-Viren MVA können auch zur Herstellung von heterologen Polypeptiden in eukaryotischen Zellen verwendet werden. Dabei werden Zellen mit den rekombinanten Vaccinia-Viren infiziert. In den Zellen wird das Gen, das für das fremde Antigen codiert, exprimiert. Das exprimierte heterologe Polypeptid kann isoliert werden. Die zur Produktion solcher heterologer Polypeptide zu verwendenden Verfahren sind dem Fachmann generell bekannt (vergl. z.B. die europäischen Patentanmeldungen mit den Publikations-Nummern 206 920 [publiziert am 30.12.1986] und 205 939 [publiziert am 30.12.1986]). Die mit Hilfe der rekombinanten MVA-Viren produzierten Polypeptide sind, auf Grund der besonderen Eigenschaften der MVA-Viren, besser für die Verwendung als Arzneimittel beim Menschen und bei Tieren geeignet.

Die Herstellung der rekombinanten Vaccinia-Viren MVA kann wie nachfolgend dargelegt durchgeführt werden. Eukaryotische Zellen, bevorzugt Säugerzellen, insbesondere CV1-Affenzellen (erhältlich von Flow Laboratories Katalog-Nr. 02-240, von der American Type Culture Collection (ATCC), 12301 Parklawn Drive, Rockville, Maryland, U.S.A., unter der Hinterlegungs-Nr. CCL 70, sowie von der European Collection of Animal Cell Cultures (ECACC), PHLS Centre for Applied Microbiology and Research, Porton Down, Salisbury, Grossbritannien, unter der Hinterlegungs-Nr. 85011434; beschrieben von Jensen et al., Proc. Natl. Acad. Sci. USA 52, 53-59 [1964]) können mit dem MVA infiziert werden. Das MVA und seine Eigenschaften sind ausführlich in der Literatur beschrieben (Mayr et al., supra). Das MVA-Virus wurde am 15.12.1987 bei CNCM (Institut Pasteur, Collection Nationale de Cultures de Microorganismes, 25, rue du Docteur Roux, 75724 Paris Cedex 15) unter der Hinterlegungsnummer I-721 gemäss den Vorschriften des Budapester Vertrags hinterlegt. Das MVA-Virus kann auf Hühnerembryo-Fibroblastzellen (HEF), wie im Beispiel beschrieben, vermehrt werden. MVA zeichnet sich durch seine starke Attenuierung aus, d.h. durch eine abgeschwächte Virulenz oder Infektionskraft unter Erhaltung einer guten Immunogenität. Das Mass der Abschwächung der Virulenz kann auf verschiedene Art und Weise experimentell bestimmt werden, z.B. durch das Verhalten des Virus auf Zellkulturen, durch Messung der Neurovirulenz oder der Virulenz bei subkutaner Applikation oder durch einen Virulenz-Test in immunsupprimierten Mäusen. Der starke attenuierte Stamm MVA zeigt eine starke Veränderung des Verhaltens auf Zellkulturen und eine mindestens um den Faktor $10^7$ geringere Neurovirulenz als der Wildtyp Vaccinia-Virusstamm WR. Dieser Unterschied zeigt sich auch beim Vergleich von rekombinanten Wildtypviren des Stammes WR mit rekombinanten Viren des Stammes MVA.

Gemäss vorliegender Erfindung wird in die infizierte Zelle eine DNA eingeführt, die eine Teilsequenz aus einem nicht-essentiellen Segment der Vaccinia-Virus-DNA, sowie eine DNA-Sequenz, die für ein fremdes Antigen codiert, enthält.

Die DNA kann durch Transfektion, z.B. mittels Kalziumphosphatpräzipitation (Graham et al., Virol. 52 456-467 [1973]; Wigler et al., Cell 16, 777-785 [1979]), mittels Elektroporation (Neumann et al., EMBO J. 1, 841-845 [1982]), durch Mikroinjektion (Graessmann et al., Meth. Enzymology 101, 482-492 [1983]), mittels Liposomen (Straubinger et al., Methods in Enzymology 101, 512-527 [1983]), mittels Sphäroplasten (Schaffner, Proc. Natl. Acad. Sci. USA 77, 2163-2167 [1980]) oder mittels weiterer, dem Fachmann bekannter Methoden in die Zellen eingeführt werden. Bevorzugt wird die Transfektion mittels Kalziumphosphatpräzipitation verwendet.

Die eingeführte DNA kann linear oder zirkulär sein. Bevorzugt wird eine zirkuläre DNA verwendet. Besonders bevorzugt ist die Verwendung eines Plasmids. Die DNA enthält mindestens eine Teilsequenz aus einem nicht-essentiellen Segment der Vaccinia-Virus-DNA. Nicht-essentielle Segmente der Vaccinia-Virus-DNA sind dem Fachmann bekannt. Ein Beispiel für ein solches nicht-essentielles Segment ist das Thymidinkinasegen und seine benachbarten Regionen (Weir et al., J. Virol., 530-537 [1983]). Die bevorzugte Teilsequenz ist im Plasmid pHGS-2/5.1 enthalten, dessen Konstruktion in der europäischen Patentanmeldung mit der Publikations-Nr. 198 328, publiziert am 22.10.1986, beschrieben ist.

Das Plasmid pHGS-2/5.1 enthält ein Gen, das für das 5.1--Antigen des Malariaerregers Plasmodium falciparum codiert (Hope et al., Nucleic Acids Res. 13, 369-379 [1985]). Anstelle dieses Gens, das für ein Malariaantigen codiert, können andere bekannte Gene aus pathogenen Agentien in der DNA enthalten sein. Unter pathogenen Agentien werden Viren, Bakterien und Parasiten verstanden, die eine Krankheit auslösen können, aber auch Tumorzellen, die sich in einem Organismus ungehemmt vermehren und daher zu krankhaften Wucherungen führen können. Beispiele solcher pathogener Agentien sind in Davis, B.D. et al., (Microbiology, 3rd. ed., Harper International Edition) beschrieben. Bevorzugte Gene von pathogenen Agentien sind solche des Malariaparasiten Plasmodium falciparum, der Tuberkulose aus lösenden Mycobakterien, von Herpes-Viren und von den Human-Immundefizienz-Viren, z.B. HIV I und HIV II.

Damit die DNA-Sequenz, bzw. das Gen, das für ein fremdes Antigen codiert, exprimiert werden kann, müssen regulatorische Sequenzen, die für die Transkription des Gens nötig sind, auf der DNA vorhanden sein. Solche regulatorischen Sequenzen (sog. Promotoren) sind dem Fachmann bekannt, so zum Beispiel diejenigen des Vaccinia 11kDa-Gens, wie sie in der Europäischen Patentanmeldung, Publikations-Nr. 198 328 beschrieben sind, sowie diejenigen des 7,5 kDa-Gens (Europäische Patentanmeldung, Publikations-Nr. 110 385).

Nachdem das für ein fremdes Antigen codierende Gen in die eukaryotische Zelle eingeführt worden ist und die fremde DNA mit der viralen DNA rekombiniert hat, kann das gewünschte rekombinante Vaccinia-Virus in an sich bekannter Weise, vorzugsweise mit Hilfe eines Markers (vergl. Nakano et al., Proc. Natl. Acad. Sci. USA 79, 1593-1596 [1982], Franke et al., Mol. Cell. Biol. 5, 1918-1924 [1985], Chakrabarti et al., Mol. Cell. Biol. 5, 3403-3409 [1985], Fathi et al., Virology 155, 97-105 [1986]), isoliert werden. Dies geschieht bevorzugt wie in den Beispielen beschrieben, indem die infizierten eukaryotischen Zellen zwei Tage nach der Transfektion auf 143B TK⁻-Zellen (ATCC Nr. CRL 8303) gegeben werden und diese in Gegenwart von Bromdeoxyuridin (BUdR) während weiterer zwei Tage inkubiert werden. Dabei werden alle Zellen, die ein funktionelles Thymidinkinasegen (TK-Gen) enthalten, getötet und nur noch die TK⁻-Zellen, sowie TK⁻-Zellen, die ein Vaccinia-Virusgenom enthalten, bei dem das TK-Gen durch Integration eines fremden Gens inaktiviert worden war, können überleben.

Dieser Selektionsschritt wird bevorzugt mehrmals durchgeführt. Um einzelne Virusklone zu erhalten, können nach der BUdR-Selektion primäre Hühnerembryo-Fibroblastzellen (HEF) mit der Zellsuspension von den 143B TK⁻ -Zellen infiziert und unter Agarosemedium, wie im Beispiel beschrieben, inkubiert werden. In den klonierten Viren kann mittels fluoreszierender Antikörper die Expression des fremden Antigens nachgewiesen werden. Positive Klone können durch Plaque-Reinigung weiter gereinigt werden. Die so erhaltenen rekombinanten Vaccinia-Viren MVA sind fähig das im viralen Genom enthaltene, für ein fremdes Antigen codierende Gen, zu exprimieren. Es hat sich gezeigt, dass die Expression solcher Gene in etwa gleich stark ist in Rekombinanten des stark attenuierten Stammes MVA, wie in Rekombinanten des üblicherweise verwendeten Stammes WR.

Zur Herstellung von Vakzinen werden die erfindungsgemässen Vaccinia-Viren MVA in eine physiologisch akzeptable Form gebracht. Dabei kann auf die langjährige Erfahrung bei der Herstellung der Vakzine, die zur Pockenimpfung verwendet wurden, abgestellt werden (Kaplan, Br. Med. Bull. 25, 131-135 [1969]). Typischerweise werden etwa $10^6$-$10^7$ Partikel des rekombinanten MVA in 100 $\mu$l phosphatgepufferter Kochsalzlösung (PBS) in Gegenwart von 2% Pepton und 1% Humanalbumin in einer Ampulle, vorzugsweise einer Glasampulle, lyophilisiert. Das Lyophilisat kann Gerüstbildner (wie Mannit, Dextran, Zucker, Glykokoll, Milchzucker oder Polyvinylpyrrolidon) oder andere Hilfsstoffe (wie Antioxidantien, Stabilisatoren, etc.) enthalten, die für eine parenterale Applikation geeignet sind. Die Glasampulle wird dann verschlossen und kann, vorzugsweise bei Temperaturen unter -20°C, mehrere Monate aufbewahrt werden.

Zur Impfung kann das Lyophilisat in 0,1 bis 0,2 ml wässriger Lösung, vorzugsweise physiologischer Kochsalzlösung, gelöst und parenteral appliziert werden, z.B. durch intradermale Inokulation. Das erfindungsgemässe Vakzin wird vorzugsweise intracutan injiziert. An der Stelle der Injektion kann eine leichte Schwellung und Rötung, manchmal auch ein Juckreiz festgestellt werden (Stickl et al., supra). Die Art der Verabreichung, die Dosierung sowie die Anzahl der Applikationen können vom Fachmann auf bekannte Art und Weise optimiert werden. Gegebenenfalls ist es zweckmässig, das Vakzin über einen längeren Zeitraum mehrmals zu applizieren, um einen hohen Titer an Antikörper gegen das fremde Antigen zu erhalten.

Das nachfolgende ausführliche Beispiel soll zum besseren Verständis der vorliegenden Erfindung beitragen. Es soll aber nicht der Eindruck erweckt werden, dass sich die Erfindung auf dem Gegenstand des Beispiels beschränkt.

Beispiel

1 Vermehrung und Reinigung der Viren

1.1 Vermehrung des MVA-Virus

Beim MVA-Virus handelt es sich um ein stark attenuiertes Vaccinia-Virus das durch serielle Passagen des Ausgangsstammes CVA auf Hühnerembryo Fibroblasten (HEF) Zellkulturen entstanden ist. Als allgemeine Uebersicht über die Entstehungsgeschichte, die Eigenschaften und die Anwendung des Vaccinia-Stammes MVA sei auf die zusammenfassende Veröffentlichung von Mayr et al. in Infection 3, 6-14 [1975] verwiesen. Durch die Adaption an HEF ist die Vermehrung des MVA-Virus auf anderen Zellsystemen stark eingeschränkt und Plaquebildung durch das Virus ist nur mehr auf HEF-Zellen nachweisbar. Obwohl keine Plaquebildung auf Affen CV1 (ATCC Hr. CCL 70) und Human 143B TK⁻-Zellen (ATCC Nr. CRL 8303; Bacchetti et al., Proc. Natl. Acad. Sci. USA 74, 1590-1594 [1977]) feststellbar ist, konnte dennoch, wie nachfolgend beschrieben, gezeigt werden, dass zumindest auf diesen beiden Zellinien eine Replikation des MVA-Virus erfolgt, die in etwa vergleichbar ist mit derjenigen auf HEF--Zellen. Die Fähigkeit des MVA-Virus auf CV1 und 143B TK⁻-Zellen zu replizieren wurde für die Rekombination fremder Antigene in das Virusgenom ausgenutzt (siehe unten). Um die Eigenschaften des MVA-Virus nicht zu verändern, erfolgte dessen Vermehrung aber normalerweise auf HEF-Zellen, der Wirtszelle für die es adaptiert worden war. Zur Herstellung der HEF-Zellen wurden 11 Tage alte Embryonen aus bebrüteten Hühnereiern isoliert, von den Extremitäten befreit, in kleine Stücke geschnitten und in einer Lösung bestehend aus 0.25% Trypsin während 2 Stunden bei Raumtemperatur langsam dissoziert. Die erhaltene Zellsuspension wurde mit einem Volumen Medium I (MEM Eagle, z.B. erhältlich von Gibco, Basel, Schweiz; Bestell-Nr. 072-1500) mit 5% fötalem Kälberserum (FCS), Penicillin (100 Einheiten/ml), Streptomycin (100 $\mu$g/ml) und 2 mM Glutamin verdünnt, durch ein Zellsieb filtriert (z.B. erhältlich von Technomara AG, Zürich, Schweiz, Bestell-Nr. Bellco 1985, 150 mesh (Maschenweite)) und die Zellen in einer Tischzentrifuge (Hermle KG, D-7209 Gosheim, BRD) durch Zentrifugation während 5 Minuten bei 2000 rpm bei Raumtemperatur sedimentiert. Das Zellsediment wurde in 1/4 des ursprünglichen Volumens Medium I aufgenommen und die so gewonnenen HEF-Zellen in Zellkulturschalen ausgesät. Je nach gewünschter Zelldichte wurden sie während 1-2 Tage in Medium I im $CO_2$ Inkubator bei 37°C wachsen gelassen und entweder direkt oder nach 1-2 weiteren Zellpassagen zur Infektion verwendet. Eine übersichtliche Beschreibung der Herstellung von Primärkulturen kann dem Buch von R.I. Freshney, "Culture of animal cells", Alan R. Liss Verlag, New York [1983], Kapitel 11, Seite 99ff entnommen werden.

Die Infektion mit MVA-Viren wurde wie folgt durchgeführt. HEF-Zellen wurden in 175 cm$^2$ Zellkulturflaschen gezüchtet. Bei 80-90%-iger Konfluenz wurde das Medium entfernt und die Zellen während einer Stunde mit einer MVA-Virussuspension (0,01 infektiöse Partikel (= Pfu) pro Zelle, 0,01 ml/cm$^2$) in phosphatgepufferter Salzlösung (PBS/Dulbecco. z.B. Amimed AG, Muttenz, Schweiz, Bestell-Nr. 23.100.10) inkubiert. Anschliessend wurde Medium I zugegeben (0.2 ml/cm$^2$) und die Flaschen bei 37°C während 2-3 Tagen inkubiert bis etwa 80% der HEF-Zellen lysiert waren. Die Viruslysate wurden vor der Weiterverarbeitung (Reinigung etc.) mit Zellen und Medium direkt in den Zellkulturflaschen bei -30°C aufbewahrt.

## 1.2 Vermehrung des WR-Virus

Im Unterschied zum MVA-Virus kann sich das WR-Virus (ATCC Nr. VR-119) in fast allen Zellinien vermehren (Drillien et al., J. Virology 28, 843-850 [1978]) und seine Vermehrung kann anhand von Plaquebildung direkt beobachtet werden. Obwohl also kein zwingender Grund bestand dieses Virus auf HEF-Zellen zu vermehren, wurden dennoch in den nachfolgend beschriebenen Versuchen, meistens diese Zellen gewählt, um, im direkten Vergleich der beiden Viren MVA und WR, wirtszellspezifische Faktoren möglichst auszuschalten. Die Vermehrung erfolgte wie für das MVA-Virus beschrieben.

## 1.3 Reinigung der Viren

Die Reinigungsschritte die unternommen wurden, um eine möglichst saubere Viruspräparation zu erhalten, die frei ist von Wirtszell-spezifischen Komponenten, waren für MVA- und WR-Virus identisch (Joklik, Virology 18, 9-18 [1962], Zwartouw et al., J. gen. Microbiol. 29, 523-529 [1962]). Die infizierten und danach bei -30°C aufbewahrten Zellkulturen wurden aufgetaut, die restlichen Zellen von der Plastikunterlage abgeschüttelt oder abgeschabt und Zellen und Virus durch Zentrifugation (Sorvall-Zentrifuge, GSA Rotor, 1 Stunde bei 5000 rpm und 10°C) vom Medium abgetrennt. Das Sediment, bestehend aus Virus- und Zellpartikeln, wurde einmal in PBS aufgeschlämmt (10-20-faches Volumen des Sediments) und die Suspension wie oben zentrifugiert. Das neue Sediment wurde in 10-fachem Volumen RSB-Puffer (10mM Tris--HCl pH 8.0, 10mM KCl, 1mM MgCl$_2$) aufgeschlämmt und die Suspension, zur Zerstörung der restlichen noch intakten Zellen und zur Befreiung der Viruspartikel aus den Zellhüllen, kurz mit Ultraschall behandelt (Labsonic 1510 ausgerüstet mit einem Stabrüssel von 4 mm Durchmesser, erhältlich von Bender und Hobein, Zürich, Schweiz; 2 mal 10 Sekunden bei 60 Watt und Raumtemperatur). Bei der anschliessenden kurzen Zentrifugation der Suspension (Sorvall Rotor GSA erhältlich von Du Pont Co., D-6353 Bad Nauheim, BRD; 3 Minuten bei 3000 rpm und 10°C) wurden die Zellkerne und die grösseren Zelltrümmern abgetrennt. Das Sediment wurde, wie oben beschrieben, nochmals in RSB-Puffer aufgeschlämmt, mit Ultraschall behandelt und zentrifugiert. Die gesammelten Ueberstände mit den freien Viruspartikeln wurden vereinigt und auf ein Kissen, bestehend aus 10 ml 35% Saccharose in 10 mM Tris-HCl pH 8,0, überschichtet und in einem Kontron TST 28.38/17 Rotor (Kontron Instrumente, Zürich, Schweiz; entspricht Beckman Rotor SW 27) zentrifugiert (90 Minuten bei 14'000 rpm und 10°C). Der Ueberstand wurde dekantiert, das Sediment mit den Viruspartikeln in 10 ml 10 mM Tris-HCl pH 8.0 aufgenommen, zur Homogenisierung kurz mit Ultraschall behandelt (2 mal 10 Sekunden bei Raumtemperatur, Gerät wie oben beschrieben) und zur weiteren Reinigung auf einen Stufengradient gegeben. Die Stufen des Gradients bestanden aus je 5 ml Saccharose in 10 mM Tris-HCl pH 8.0 (Saccharose-Konzentrationsstufen:20%, 25%, 30%, 35% und 40%). Der Gradient wurde in einem Kontron TST 28.38/17 Rotor während 35 Minuten bei 14'000 rpm und 10°C zentrifugiert. Nach dieser Zentrifugation waren mehrere diskrete Zonen, die Viruspartikel enthalten, im Gradienten-Bereich zwischen 30% und 40% Saccharose sichtbar. Dieser Bereich wurde aus dem Gradient abgezogen (10 ml), die Saccharoselösung mit PBS verdünnt (20 ml) und die Viruspartikel durch Zentrifugation daraus sedimentiert (Kontron TST 28.38/17 Rotor, 90 Minuten bei 14'000 rpm. 10°C). Das Sediment, das jetzt grösstenteils aus reinen Viruspartikeln bestand (Vergleich zwischen OD-Messung und Plaquetest, siehe unten), wurde in PBS aufgenommen und zwar so, dass die Viruskonzentrationen durchschnittlich 1-5 x 10$^9$ Pfu/ml entsprach. Die gereinigte Virus-Stocklösung wurde entweder direkt, oder mit PBS verdünnt, für die weiteren Versuche eingesetzt.

## 1.4 Konzentrationsbestimmung

Die Konzentrationsbestimmung der Viruspräparationen erfolgte gewöhnlich auf zwei Arten und erlaubte so auch eine Aussage über die Reinheit des Virusstocks. Durch Messen der optischen Dichte der Viruslösung in einem Spektrophotometer bei 260 nm ($OD_{260}$) liess sich die absolute Konzentration an Viruspartikel in der Lösung bestimmen, wobei 1 $OD_{260}$ einer Viruskonzentration von $1.2 \times 10^{10}$ Partikel pro ml entspricht (Joklik, supra). Dazu wurden die obigen Viruslösungen mit PBS im Verhältnis 1:100 verdünnt und die Absorption in Quarzküvetten gegen PBS-Lösung als Nullwert gemessen. Unter der Annahme, dass nur etwa jedes sechzigste Viruspartikel aus einer solchen Präparation Zellkultur--Zellen infizieren kann, also infektiös ist (Joklik, Bacteriol. Rev. 30, 33-66 [1966]), können der, aus der optischen Dichtemessung berechnete Virustiter und der, nach der nachfolgend beschriebenen Methode, erhaltene Virustiter direkt miteinander verglichen werden.

Zum Austitrieren der Viruslösungen auf Zellkulturen wurden HEF-Zellkulturen in 8 cm$^2$ Plastik-Zellkultur-schalen in Medium I vermehrt. Von zu 80-90% konfluenten Schalen wurde das Medium abgesaugt und je 0.2 ml mit PBS verdünntes Virus zugegeben. Danach wurden die Schalen während 1 Stunde bei Raumtemperatur stehengelassen. Die Verdünnung der Viruslösung erfolgte in Zehnerschritten. Nach dieser Inkubation bei Raumtemperatur wurde zu jeder Schale 2 ml Agarosemedium I zugegeben (bestehend aus Medium I + 1% Agarose) und die Schalen während 16-24 Stunden im $CO_2$ Inkubator inkubiert. Danach wurden 2 ml Agarosemedium I, das zur Anfärbung der lebenden Zellen zusätzlich 0.2% Neutralrot enthielt, überschichtet und die Schalen während 16-24 Stunden weiter inkubiert. Nach Ablauf dieser Zeit konnten die farblosen Plaques unter einem Binokular ausgezählt und die erhaltene Anzahl Plaques mit dem, aus der optischen Dichte berechneten Wert, verglichen werden. In den meisten Fällen wurde eine gute Uebereinstimmung gefunden. Der aus dem optischen Messwert errechnete Virustiter lag meist um etwa einen Faktor 2 bis 4 höher.

1.5 Vermehrung des MVA-Virus in verschiedenen Zellinien

### Tabelle I

| | 1/500 | 1/50 | 1/5 | 1:20 | 1:100 | 1:500 |
|---|---|---|---|---|---|---|
| LM TK⁻ | ++ | ++ | +++ | 5/9 | 0/0 | 0/0 |
| RK13 | + | + | ++++ | 10/13 | 3/3 | 1/0 |
| CV1 | ++ | +++ | +++++ | nb | 500/nb | 110/140 |
| RITA | ++ | +++++ | ++++++ | 31/38 | 10/5 | 3/0 |
| HeLa | ++ | ++ | +++++ | 31/39 | 10/9 | 1/0 |
| AG1523 | +++++ | +++++ | ++++++ | 11/8 | 1/0 | 0/0 |
| 143B TK⁻ | ++ | +++++ | ++++++ | 156/nb | 60/86 | 30/29 |
| | | | | 25 | 5 | 1 |
| | | | | Zugegebene Virusmenge | | |

(nb = nicht bestimmt)

Die in der Tabelle I aufgeführten Zellinien LM TK⁻ (ATCC Nr. CCL 1.3), RK13 (ATCC Nr. CCL 37), CV1 (ATCC Nr. CCL 70), Rita (RC-37, Schröder et al., J. gen. Virol. 41, 493-501 [1978]), HeLa (ATCC Nr. CCL 2). AG1523 (Human Genetic Mutant Cell Repository, National Institute of General Medical Science NIGMS, Camden, New Jersey, USA) und 143B TK⁻ (ATCC Nr. CRL 8303) wurden in 8 cm$^2$ Schalen ausgesät (je 12 Schalen pro Linie) und unter Medium I bis zu 80-90% Konfluenz bei 37°C im $CO_2$ Inkubator wachsengelassen. Anschliessend wurde das Medium abgesogen und die Zellen mit je 0.2 ml MVA-Virus (in PBS) infiziert und zwar in Konzentrationen von einem infektiösen Viruspartikel (Pfu) pro 500, 50 und 5 Zellen. Nach 1 Stunde bei Raumtemperatur wurden 2 ml Medium I auf die Zellen gegeben und diese bei 37°C inkubiert. Nach 2 Tagen wurde die Hälfte der Kulturschalen vom Medium befreit und die Zellen mit einer Lösung von 2% Neutralrot in 18% Aethanol (1 ml/Schale) fixiert und angefärbt. In keiner der angefärbten Kulturen konnten Plaques nachgewiesen werden, aber die Zellen jeder Kultur zeigten zytopathische Effekte (Zellveränderungen), die je nach Zellinie stark (+ + + +) oder schwach (+) sein konnten. Hohe Viruskonzentrationen (1 Pfu/5 Zellen) führten in den meisten Fällen zum allgemeinen Zelltod (+ + + + + +). Siehe linke Hälfte von Tabelle I.

Aus dem oben beschriebenen Versuch liess sich nicht ablesen, ob eine Vermehrung des MVA Virus in den verschiedenen Zellinien stattgefunden hat oder nicht. Daher wurden jeweils das Medium und die Zellen aus den restlichen, dicht angefärbten Kulturschalen gesammelt, durch Ultraschall kurz homogenisiert und die so gewonnenen Homogenate für die Infektion von HEF-Zellen verwendet. Die Homogenate wurden im Verhältnis 1:20, 1:100 und 1:500 mit PBS verdünnt. Je 0,2 ml dieser Verdünnungen wurden auf je 2 Schalen HEF-Zellen, die zu 80-90% konfluent waren und von denen zuvor das Medium entfernt worden war, gegeben. Die Zellen wurden während einer Stunde bei Raumtemperatur inkubiert und danach mit Agarosemedium I überschichtet (2 ml/Schale). Nach 16-24 stündiger Inkubation bei 37°C wurde Agarosemedium I mit 0.2% Neutralrot zugegeben (2 ml/Schale) und die Schalen während weiterer 16-24 Stunden inkubiert. Nun waren die farblosen Plaques gut sichtbar und wurden gezählt (Pfu/Schale). In der rechten Hälfte von Tabelle I sind die Ergebnisse für die einzelnen Zellinien festgehalten. Es handelt sich dabei um die Werte für die Verdünnungen der mittleren Viruskonzentrationen (1 Pfu/50 Zellen). Aus der ursprünglich eingesetzten Anzahl von MVA-Viruspartikel (Pfu/Zelle), der Verdünnung der Homogenate und dem für die Infektion der HEF-Zellen verwendeten Volumen lässt sich die Anzahl Viruspartikel errechnen, die auf die HEF aufgebracht wurden (=zugegebene Virusmenge), vorausgesetzt das MVA-Virus hätte sich in den einzelnen Zellinien nicht vermehrt. Aus der Tabelle geht hervor, dass die Anzahl der Plaques nur in zwei der getesteten Zellinien (CV1 und 143B TK⁻) signifikant über dem Wert liegt, der auf Grund der zugegebenen Virusmenge erwartet wird, d.h. nur in diesen beiden Zellinien hat eine messbare Vermehrung des MVA-Virus stattgefunden.

2 Einbringen eines fremden Antigens

2.1 Plasmid mit Malaria-5.1-Antigen

Das Einbringen des fremden Antigens in das Vaccinia Virus Genom erfolgte gemäss der von Mackett et al., (Proc. Natl. Acad. Sci. USA 79, 7415-7419 [1982]) beschriebenen Methode und basiert auf homologen Rekombinationen im viralen TK-Gen. Die Konstruktion des Plasmids pHGS-2/5.1. das im vorliegenden Beispiel für die Rekombination verwendet wurde, ist in der Europäischen Patentanmeldung, Publikations-Nr. 198 328, publiziert am 22.10.1986 ausführlich beschrieben. Das Ausgangsplasmid pHGS-2 wurde in Form einer Kultur von E.coli HB101 transformiert mit dem Plasmid pHGS-2 am 21.2.1985 unter der Hinterlegungsnummer DSM 3249 bei der Deutschen Sammlung von Mikroorganismen DSM in Göttingen. Deutschland in Uebereinstimmung mit dem Budapester Vertrag hinterlegt und am 4.10.1986 frei zugänglich gemacht. Seit November 1987 ist die DSM in D-3300 Braunschweig am Mascheroder Weg 1B untergebracht.

2.2 Rekombination in das WR-Virus

Die Rekombination des 5.1-Antigens in das WR-Virus erfolgte nach einem Protokoll das eine zweifache Selektion auf rekombinante Viren erlaubt. In einem ersten Schritt wurden 8 cm² Kulturschalen mit CV1-Affenzellen, die zu 80-90% konfluent waren, vom Medium befreit und anschliessend mit je 0.2 ml einer Virussuspension der temperatursensitiven Vaccinia Mutante ts N7 (Drillien et al., Virology 131, 385-393 [1983]) überschichtet (0.1 Pfu/Zelle). Die Schalen wurden 1 Stunde bei Raumtemperatur stehen gelassen. Anschliessend wurde zu den infizierten Zellen Medium I zugegeben (2 ml/Schale) und die Schalen während 2 Stunden bei der für das Virus permissiven Temperatur von 33°C im $CO_2$ Brutschrank inkubiert. (Kieny et al., Nature 312, 163-166 [1984]). Eine halbe Stunde vor Ablauf dieser Inkubationszeit wurde ein, die DNAs enthaltender, Kalziumphosphat Niederschlag vorbereitet (Graham et al., supra). Dazu wurden zu einer Vorlage von 0.55 ml HeBS-Puffer (1,5 mM Dinatriumhydrogenphosphat, 280 mM NaCl, 50 mM HEPES, eingestellt auf pH 7.1), 200 ng gereinigte Vaccinia-WR-Virus-DNA und 200 ng der gereinigten Plasmid-DNA pHGS-2/5.1 in je 1 µl TE-Puffer (10 mM Tris-HCl pH 7.5, 1 mM EDTA) zugegeben. Unter leichtem Bewegen wurden 0.55 ml einer 250 mM Kalziumchloridlösung zugetropft und die Mischung während 20 Minuten bei Raumtemperatur stehen gelassen. Anschliessend wurde das Medium von den Kulturschalen abgezogen und die Zellen mit je 0.25 ml des Präzipitats mit den DNAs versetzt. Nach einer einstündigen Inkubation bei Raumtemperatur wurden je 2 ml Medium I zugegeben und die Zellen während 2 Stunden bei 39.5°C im $CO_2$ Schrank inkubiert (Kieny et al., supra). Bei dieser erhöhten Temperatur können sich die ursprünglich eingesetzten ts N7-Viren nicht mehr vermehren, was eine Selektion für Viren erlaubt, die zumindest Rekombinationen im ts7 Locus eingegangen sind. Da sich das auf den Zellen liegende Kalziumphosphat auf die Dauer nachträglich auf das Zellwachstum auswirkte, wurde nach zweistündiger Inkubation das Medium entfernt, die Zellen 3 mal mit je 1 ml PBS unter leichtem Bewegen gewaschen, die PBS-Lösung jeweils abgesogen, wieder je 2 ml des Mediums I auf die Zellen gegeben und diese weitere 2 Tage bei 39.5°C im $CO_2$ Inkubator gehalten. Danach wurden die Kulturschalen mit den Zellen und dem Medium bei -30°C eingefroren, wieder aufgetaut, die noch haftenden Zellen von den Schalen abgekratzt und die Suspension wie oben beschrieben kurz mit Ultraschall behandelt. Von diesem Homogenat wurden für den zweiten Selektionsschritt je 0.2 ml unverdünnt, oder 1:5 bzw. 1:30 mit PBS verdünnt, auf einen beinahe konfluenten Rasen von Human 143B TK⁻-Zellen in 8 $cm^2$ Kulturschalen, von denen zuvor das Medium entfernt worden war, aufgebracht. Die infizierten Zellen wurden während 1 Stunde bei Raumtemperatur gehalten und anschliessend mit je 2 ml Agarosemedium II (Medium I plus Nichtessentielle-Aminosäurenlösung [Gibco; Bestell-Nr. 043-1140] plus Vitamine [Gibco; Bestell-Nr. 043-1120] plus 1% Agarose) enthaltend 0.1 mg/ml Bromdeoxyuridin (BUdR), überschichtet. Danach wurden die Schalen während 16-24 Stunden bei 37°C im $CO_2$ Inkubator inkubiert. Eine zweite Schicht Agarosemedium II, enthaltend zusätzlich noch 0.2% Neutralrot wurde aufgebracht und die Schalen während weiterer 16-24 Stunden inkubiert. Nach dieser Zeit waren die farblosen Plaques normalerweise gut sichtbar und das darin enthaltende Virus konnte in Form eines Agarosezylinders als einzelner Klon mit einer Pasteurpipette ausgestochen werden (Plaque-Reinigung). Auf diese Art ausgestochene Klone wurden auf CV1-Zellen wie üblich vermehrt und nach der Vermehrung einer zweiten und dritten Plaque-Reinigung unterzogen. Die abschliessende Vermehrung und Reinigung des so erhaltenen rekombinanten Virus WR 5.1 erfolgte wie oben beschrieben.

## 2.3 Rekombination in das MVA-Virus

Wie oben angesprochen und in Tabelle I dargelegt ist das MVA-Virus, obwohl keine Plaquebildung auftritt, in der Lage sich in CV1- und 143B TK⁻-Zellen zu vermehren. Diese Tatsache erleichtert eine Rekombination von fremden Antigenen in das TK-Gen des MVA-Virus in zweifacher Hinsicht. Einerseits hat es sich in den Versuchen herausgestellt, dass CV1--Zellen ein besserer Wirt für den Rekombinationsprozess abgeben als HEF. Zum andern ist, durch die Replikation des Virus in 143B TK⁻-Zellen, auch die Selektion auf TK⁻-Virus vereinfacht worden.

Die Rekombination fremder Antigene in das MVA-Virus unterscheidet sich im wesentlichen in zwei Punkten von der für das WR-Virus beschriebenen Methode. Zum einen konnte, um die Struktur des MVA-Virus, ausser im TK-Gen, nicht zu verändern, die Selektion über das temperatursensitive Virus ts N7 nicht angewendet werden. Andererseits wurden bei der BUdR-Selektion die 143B TK⁻-Zellen nicht mit Agarosemedium II sondern mit normalem Medium II (Medium I plus Nichtessentielle-Aminosäurenlösung [Gibco, Bestell-Nr. 043-1140] plus Vitamine [Gibco; Bestell-Nr. 043-1120]) versetzt und das Ausstechen von einzelnen Plaques erfolgte erst nach einer Infektion von HEF mit dem selektierten TK⁻-Virus und Inkubation unter Agarosemedium I.

Das für das MVA-Virus angewendete Protokoll der Rekombination entspricht annähernd dem von Mackett et al., (Proc. Natl. Acad. Sci. USA 79, 7415-7419 [1982]) für das WR-Virus beschriebenen Verfahren. CV1-Zellen auf 8 $cm^2$ Kulturschalen wurden bei 80-90%iger Konfluenz, vom Medium befreit, mit je 0.2 ml einer MVA-Virussuspension überschichtet (0.1 Pfu/Zelle) und während 1 Stunde bei Raumtemperatur inkubiert. Danach wurden je 2 ml Medium I zu den infizierten Zellen pipettiert und die Zellen bei 37°C im $CO_2$ Brutschrank während 2 Stunden inkubiert. Die folgenden Schritte entsprechen dem für das WR-Virus angewendeten Verfahren (siehe oben), ausser dass für das Kalziumphosphat-Präzipitat keine WR-Virus-DNA zugegeben wurde, die nachfolgende Inkubation der Zellen bei 37°C erfolgte und die 143B TK⁻-Zellen mit 2 ml Medium II ohne Agarose aber mit 0.1 mg/ml BUdR überschichtet wurden. Nach zweitägiger Inkubation wurden die Kulturschalen für kurze Zeit auf -30°C abgekühlt, die Lösung wieder aufgetaut, die restlichen Zellen von der Unterlage abgekratzt und Zellen und Medium durch kurze Ultraschallbehandlung wie oben beschrieben homogenisiert. Nach Entfernen des Mediums aus 8 $cm^2$ Kulturschalen, die zu 80-90% mit HEF überwachsen waren, wurden verschiedene Verdünnungen dieses Homogenats auf die Zellen gegeben (0.2 ml/Schale) und die Infektion 1 Stunde bei Raumtemperatur gehalten. Anschliessend wurden die HEF mit 2 ml Agarosemedium I überschichtet, 16-24 Stunden bei 37°C inkubiert und danach eine zweite Schicht Agarosemedium I (2 ml), das ausserdem 0.2% Neutralrot enthielt, darauf gegeben. Nach weiteren 16-24 Stunden waren die farblosen Plaques gut sichtbar und das darin enthaltene Virus konnte mittels einer Pasteurpipette zusammen mit der darüberliegenden Agarose ausgestochen werden. Der so isolierte Virus-Klon wurde auf CV1-Zellen vermehrt und, zur Reinigung des Virus-Klons, die Selektion auf den 143B TH⁻-Zellen sowie die daran anschliessenden Schritte 1-2 mal wiederholt (Plaque-Reinigung). Die abschliessende Vermehrung und Reinigung des so erhaltenen rekombinanten Virus MVA 5.1 erfolgte in der weiter oben beschriebenen Art und Weise.

3 Test auf Expression des 5.1-Antigens

3.1 In vitro Expression in Zellkulturen

Um die Expression des Malaria-5.1-Ag durch die rekombinanten Viren WR 5.1 und MVA 5.1 in CV1-Zellen zu testen, wurden mit dem rekombinanten Virus infizierte CV1-Zellen durch kurzes Zentrifugieren in einer Tischzentrifuge (Hettich Mikrorapid K, erhältlich von A. Hettich AG, Bäch, Schweiz; max. Geschwindigkeit während 3 Minuten bei 20°C) sedimentiert, das Sediment zweimal mit PBS gewaschen, in PBS aufgenommen und auf einen Mikroskop-Objektträger aus Glas aufgebracht und dort eintrocknen gelassen. Ein anderes Verfahren bestand darin CV1-Zellen direkt auf einem Objektträger zu züchten, mit dem Virus zu infizieren, die so infizierten Zellen nach 1-2 Tagen mit PBS vom Inkubationsmedium freizuwaschen und bei Raumtemperatur auf dem Glasträger eintrocknen zu lassen. Zur Fixierung der Zellen wurden die Objektträger während mindestens 1 Stunde bei -30°C in Aceton inkubiert und anschliessend bei Raumtemperatur trocknen gelassen.

In PBS verdünntes Kaninchen-anti-5.1-Antiserum wurde so auf die vorbereiteten Objektträger aufgebracht, dass die Zellen mit einer Flüssigkeitsschicht bedeckt waren. Die Objektträger wurden in einer feuchten Hammer während 1 Stunde bei 37°C inkubiert, danach mehrmals mit PBS gewaschen und ohne sie trocknen zu lassen der zweite, in PBS verdünnte, Fluoresceinisothiocyanat (FITC) markierte Antikörper (Ziege-anti-Kaninchen-IgG, Nordic GAR/Ig/FITC, erhältlich von Biogenzia Lemania SA, Lausanne, Schweiz) auf die Zellen gegeben. Nach einstündiger Inkubation bei 37°C in einer feuchten Hammer wurden die Objektträger mehrmals mit PBS gewaschen und gut trocknen gelassen. Die Zell-Präparate konnten danach im Mikroskop unter UV-Licht auf Fluoreszenz-Strahlung getestet werden.

3.2 In vivo Expression in Mäusen

Um die Expression des Malaria-5.1-Antigens durch das rekombinante MVA-Virus mit derjenigen des rekombinanten WR-Virus in vivo vergleichen zu können, wurde die anti-5.1-Antikörperproduktion in Mäusen, die mit diesen rekombinanten Viren immunisiert worden waren, gemessen. Dazu wurden Swiss Albino Mäuse subkutan zweimal, im Abstand von 2 Wochen, entweder mit der 5.1-Rekombinante des WR-Virus ($10^8$ Pfu/Maus) oder der 5.1-Rekombinante des stark attenuierten MVA-Virus ($10^9$ Pfu/Maus) gespritzt. 2 Wochen nach der letzten Injektion wurde nach Abschneiden der Schwanzspitze etwas Blut in eine heparinisierte Kapillare aufgesogen (10 $\mu$l/Maus), die Kapillare in 90 $\mu$l PBS ausgeblasen und aus dieser Lösung durch Zentrifugation die Serumfraktion gewonnen. Verschiedene Verdünnungsreihen des Serums wurden in ELISA-Tests (Voller et al.. The enzyme linked immunosorbent assay (ELISA), Dynatech [1979], erhältlich von Dynatech Produkte AG, Embrach, Schweiz) auf die Menge der darin enthaltenen, gegen das Malaria-5.1-Antigen gerichteten, Antikörper getestet. Dazu wurden Multititer-Platten (z.B. Dynatech Produkte AG; Bestell-Nr. 655001) mit 0,25 $\mu$g/Vertiefung, aus E.coli isoliertem, rekombinantem 5.1-Antigen (Hope et al., Nature 308, 191-194 [1984]) beschichtet. Die Resultate eines solchen vergleichenden Versuchs sind in Tabelle II zusammengestellt.

## Tabelle II

### Antikörperproduktion

#### anti-5.1-Antigen ELISA-Titer

a)  WR 5.1       ($10^8$ Pfu/Maus)

1:15.000  
1:31.000  
1:30.000      }   1:22.800  
1:30.000  
1: 8.000

**WR WT**               1:200

b)  MVA 5.1       ($10^9$ Pfu/Maus)

1: 6.000  
1: 6.500  
1: 8.000      }   1:6.700  
1: 7.100  
1: 6.000

**MVA WT**            1:250

Je fünf 3 Wochen alte Swiss Albino Mäuse wurden wie oben beschrieben a) mit dem rekombinanten Vaccinia-Virus WR (WR 5.1) oder b) mit dem rekombinanten Vaccinia-Virus MVA (MVA 5.1) immunisiert und die Titer an anti-5.1-Antikörper im Serum durch ELISA bestimmt. Als Kontrollimpfung wurden zusätzlich je 2 Mäuse mit der entsprechenden Menge der Ausgangsviren (WR WT bzw. MVA WT) gespritzt. Während in den Kontrollseren ein Titer von 1:200 (WR WT) bzw. 1:250 (MVA WT) gemessen wurde, lagen die Werte in den Seren, der, mit den rekombinanten Viren behandelten Tiere, im Durchschnitt um einen Faktor 30 (MVA 5.1) bzw. 120 (WR 5.1) über dem Wert der Kontrollseren.

4 Virulenzverhalten des rekombinierten MVA-Virus

4.1 Verhalten in Zellkulturen

Das rekombinante MVA-Virus (MVA 5.1) bildet, im Gegensatz zum Ausgangsvirus (MVA WT), auf CV1-Zellen Plaques. Es machte also den Anschein, als hätte sich das Verhalten des rekombinanten MVA-Virus in Zellkulturen im Vergleich zum MVA Ausgangsvirus leicht verändert. Da diese Veränderung nicht auf eine Verunreinigung durch andere Virusstämme zurückzuführen war, lag der Verdacht nahe, dass, durch die Rekombination des 5.1-Antigens in das virale TK-Gen, eine Zunahme der Virulenz oder ein unterschiedliches Wirt-Virus-Verhalten eingetreten sein könnte.

4.2 Neurovirulenz in Mäusen

Das leicht veränderte Verhalten des rekombinanten MVA-Virus in Zellkulturen, nämlich das Bilden von Plaques auf CV1-Zellen, liess die Frage nach einer möglichen Steigerung der Virulenz, hervorgerufen durch das Einbringen der fremden DN; in das virale Thymidinkinase-Gen, aufkommen. Eine Möglichkeit dieser Frage nachzugehen war, die Neurovirulenz der Viren zu messen. Dazu wurde der $LD_{50}$ Wert nach intracerebraler Injektion der Viren in 1 bis 2 Tage alte Mäuse bestimmt. Zuerst wurden die Konzentrationen aller fünf getesteten Viren mit PBS auf eine Ausgangs-Konzentration von $4 \times 10^9$ Pfu/ml eingestellt. Davon ausgehend wurden 10-fach-Verdünnungen in PBS hergestellt, bis zu einer Konzentration von $4 \times 10^2$ Pfu/ml. Die verwendeten Viren waren das WR-Virus (ATCC Nr. VR-119 = WR WT), das darauf basierende rekombinante WR 5.1-Virus (WR 5.1), das Elstree-Virus (ATCC Nr. VR-862 = LS WT), das MVA-Ausgangsvirus (MVA WT), sowie seine 5.1-Rekombinante (MVA 5.1). 1-2 Tage alte Mäuse (Swiss Albino) wurden in Gruppen zu 5 Mäusen eingeteilt und je 2 solche Gruppen in der Obhut einer Muttermaus in einem Käfig belassen. Je Virusart und Viruskonzentration wurden 5 Mäusen 10 µl der Virussuspension in die linke Hirnhälfte gespritzt und das Verhalten der Tiere über einen Zeitraum von zwei Wochen, bzw. bis zu ihrem Ableben beobachtet. Aus der Ueberlebensrate der Mäuse in den einzelnen Gruppen wurde mittels der Formel von Reed et al. (Amer. J. Hyg. 27. 493-497 [1938]) der $LD_{50}$ Wert für eine Virusart errechnet. Die Ergebnisse dieser Versuche sind in Tabelle III zusammengestellt. Es zeigt sich, dass durch den Rekombinationsvorgang in das MVA-Virus keine Zunahme der Pathogenität des Virus, zumindest betreffs der Neurovirulenz eingetreten ist und dass ein grosser Unterschied in der Neurovirulenz der MVA-Viren (MVA WT und MVA 5.1) gegenüber den anderen Vaccinia-Viren besteht.

Tabelle III

| Neurovirulenz in Mäusen | |
|---|---|
| | Log $LD_{50}$ |
| WR WT | = -8.7 |
| WR 5.1 | = -7.7 |
| LS WT | = -5.8 |
| MVA WT | = -1.6 |
| MVA 5.1 | = -1.5 |

**Patentansprüche**
**Patentansprüche für folgende Vertragsstaaten : AT, BE, CH, DE, FR, GB, IT, LI, LU, NL, SE**

1. Ein Vaccinia-Virus MVA, enthaltend eine DNA-Sequenz, die für ein fremdes Antigen codiert.

2. Ein Vakzin enthaltend ein rekombinantes Vaccinia-Virus MVA gemäss Anspruch 1.

3. Ein rekombinantes Vaccinia-Virus MVA gemäss Anspruch 1 als ein Vakzin.

4. Ein Verfahren zur Herstellung eines rekombinanten Vaccinia-Virus gemäss Anspruch 1, dadurch gekennzeichnet, dass
   (a) eukaryotische Zellen mit dem Vaccinia-Virus MVA infiziert werden,
   (b) in die infizierten Zellen eine DNA eingeführt wird, die innerhalb einer Teilsequenz aus einem nicht-essentiellen Segment der Vaccinia-Virus-DNA eine DNA-Sequenz enthält, die für ein fremdes Antigen codiert und
   (c) das rekombinante Vaccinia-Virus MVA in an sich bekannter Weise isoliert wird.

5. Ein Vakzin enthaltend ein Vaccinia-Virus MVA gemäss Anspruch 1 zur Prophylaxe einer Infektion, die durch ein pathogenes Agens verursacht wird.

6. Verwendung eines rekombinanten Vaccinia-Virus MVA gemäss Anspruch 1 zur Herstellung eines Vakzins zur Prophylaxe einer Infektion, die durch ein pathogenes Agens, das das fremde Antigen enthält, verursacht wird.

7. Verwendung eines rekombinanten Vaccinia-Virus MVA gemäss Anspruch 1 zur Herstellung eines heterologen Proteins in eukaryotischen Zellen.

**Patentansprüche für folgende Vertragsstaat : ES**

1. Ein Verfahren zur Herstellung eines rekombinanten Vaccinia-Virus MVA, dadurch gekennzeichnet, dass
(a) eukaryotische Zellen mit dem Vaccinia-Virus MVA infiziert werden,
(b) in die infizierten Zellen eine DNA eingeführt wird, die innerhalb einer Teilsequenz aus einem nicht-essentiellen Segment der Vaccinia-Virus-DNA eine DNA-Sequenz enthält, die für ein fremdes Antigen codiert und
(c) das rekombinante Vaccinia-Virus MVA in an sich bekannter Weise isoliert wird.

2. Ein Verfahren zur Herstellung eines Vakzins enthaltend ein rekombinantes Vaccinia-Virus MVA, dadurch gekennzeichnet, dass man ein rekombinantes Vaccinia-Virus MVA gemäss Anspruch 1 in eine für Injektionszwecke geeignete Form bringt.

3. Verwendung eines rekombinanten Vaccinia-Virus MVA gemäss Anspruch 1 zur Herstellung eines Vakzins zur Prophylaxe einer Infektion, die durch ein pathogenes Agens, das das fremde Antigen enthält, verursacht wird.

4. Verwendung eines rekombinanten Vaccinia-Virus MVA gemäss Anspruch 1 zur Herstellung eines heterologen Proteins in eukaryotischen Zellen.

**Claims**

**Claims for the following Contracting States : AT, BE, CH, DE, FR, GB, IT, LI, LU, NL, SE**

1. An MVA vaccinia virus containing a DNA sequence which codes for a foreign antigen.

2. A vaccine containing a recombinant MVA vaccinia virus according to claim 1.

3. A recombinant MVA vaccinia virus according to claim 1 as a vaccine.

4. A method for the preparation of a recombinant vaccinia virus according to claim 1, characterized by
(a) infecting eukaryotic cells with the MVA vaccinia virus,
(b) introducing into the infected cells a DNA which contains, within a partial sequence from a non-essential segment of the vaccinia virus DNA, a DNA sequence which codes for a foreign antigen and
(c) isolating the recombinant MVA vaccinia virus in a manner known per se.

5. A vaccine containing an MVA vaccinia virus according to claim 1 for the prophylaxis of an infection which is caused by a pathogenic agent.

6. The use of a recombinant MVA vaccinia virus according to claim 1 for the preparation of a vaccine for the prophylaxis of an infection which is caused by a pathogenic agent containing the foreign antigen.

7. The use of a recombinant MVA vaccinia virus according to claim 1 for the preparation of a heterologous protein in eukaryotic cells.

**Claims for the following Contracting State : ES**

1. A method for the preparation of a recombinant MVA vaccinia virus, characterized by
(a) infecting eukaryotic cells with the MVA vaccinia virus,
(b) introducing into the infected cells a DNA which contains, within a partial sequence from a non-essential segment of the vaccinia virus DNA, a DNA sequence which codes for a foreign antigen and
(c) isolating the recombinant MVA vaccinia virus in a manner known per se.

2. A method for the preparation of a vaccine containing a recombinant MVA vaccinia virus, characterized by bringing a recombinant MVA vaccinia virus according to claim 1 into a form suitable for injection purposes.

3. The use of a recombinant MVA vaccinia virus according to claim 1 for the preparation of a vaccine for the prophylaxis of an infection which is caused by a pathogenic agent containing the foreign antigen.

4. The use of a recombinant MVA vaccinia virus according to claim 1 for the preparation of a heterologous protein in eukaryotic cells.

**Revendications**
**Revendications pour les Etats contractants suivants : AT, BE, CH, DE, FR, GB, IT, LI, LU, NL, SE**

1. Virus de la vaccine MVA contenant une séquence d'ADN qui code pour un antigène étranger.

2. Vaccin contenant un virus de la vaccine MVA recombinant selon la revendication 1.

3. Virus de la vaccine MVA recombinant selon la revendication 1, en tant que vaccin.

4. Procédé pour la production d'un virus de la vaccine recombinant selon la revendication 1, caractérisé en ce que
   (a) on infecte des cellules eucaryotes avec le virus de la vaccine MVA,
   (b) on introduit dans les cellules infectées un ADN qui, à l'intérieur d'une séquence partielle à base d'un segment non essentiel de l'ADN du virus de la vaccine, contient une séquence d'ADN qui code pour un antigène étranger, et
   (c) on isole d'une façon connue en soi le virus de la vaccine MVA recombinant.

5. Vaccin contenant un virus de la vaccine MVA selon la revendication 1, pour la prophylaxie d'une infection qui est provoquée par un agent pathogène.

6. Utilisation d'un virus de la vaccine MVA recombinant selon la revendication 1, pour la préparation d'un vaccin destiné à la prophylaxie d'une infection qui est provoquée par un agent pathogène qui contient l'antigène étranger.

7. Utilisation d'un virus de la vaccine MVA recombinant selon la revendication 1, pour la production d'une protéine hétérologue dans des cellules eucaryotes.

**Revendications pour l'Etat contractant suivant : ES**

1. Procédé pour la production d'un virus de la vaccine MVA recombinant, caractérisé en ce que
   (a) on infecte des cellules eucaryotes avec le virus de la vaccine MVA,
   (b) on introduit dans les cellules infectées un ADN qui, à l'intérieur d'une séquence partielle à base d'un segment non essentiel de l'ADN du virus de la vaccine, contient une séquence d'ADN qui code pour un antigène étranger, et
   (c) on isole d'une façon connue en soi le virus de la vaccine MVA recombinant.

2. Procédé pour la préparation d'un vaccin contenant un virus de la vaccine MVA recombinant, caractérisé en ce que l'on met sous une forme appropriée à des fins d'injection un virus de la vaccine MVA recombinant selon la revendication 1.

3. Utilisation d'un virus de la vaccine MVA recombinant selon la revendication 1, pour la préparation d'un vaccin pour la prophylaxie d'une infection qui est provoquée par un agent pathogène qui contient l'antigène étranger.

4. Utilisation d'un virus de la vaccine MVA recombinant selon la revendication 1, pour la production d'une protéine hétérologue dans des cellules eucaryotes.